# EUROPEAN PATENT APPLICATION

(11) **EP 4 696 788 A1**
(43) Date of publication of application: **18.02.2026**
(21) Application number: 23932770.3
(22) Date of filing: 08.11.2023
(51) Int. Cl.: C21B 7/00, C21C 5/38, C21C 5/40, C07C 67/36, C07C 69/36, C07C 29/149, C07C 31/20

(54) **BLAST FURNACE LONG-PROCESS STEEL-CHEMICAL CO-PRODUCTION PROCESS HAVING NEAR-ZERO CARBON EMISSION AND SYSTEM THEREOF**

(30) Priority: 11.04.2023 CN 202310381541
(71) Applicant: CISDI Engineering Co., Ltd, Chongqing 400013 (CN); CISDI Shanghai Engineering Co., Ltd., Shanghai 200940 (CN)
(72) Inventor: WANG, Gang, Chongqing 400013 (CN); HE, Xuekun, Chongqing 400013 (CN); ZHAO, Yunjian, Chongqing 400013 (CN); LI, Peng, Chongqing 400013 (CN); FAN, Xuefeng, Chongqing 400013 (CN); HE, Kun, Chongqing 400013 (CN); NIU, Qun, Chongqing 400013 (CN); HONG, Zhibin, Chongqing 400013 (CN); LAI, Feifei, Chongqing 400013 (CN); LI, Muming, Chongqing 400013 (CN); LONG, Meng, Chongqing 400013 (CN)
(74) Representative: Chung, Hoi Kan
(86) International application number: PCT/CN2023/130323
(87) International publication number: WO 2024/212499

(57) **Abstract**

The present invention discloses a near-zero carbon emission integrated process and system for long-process blast furnace steelmaking and chemical co-production. The process comprises the following steps: subjecting blast furnace gas to pressure swing adsorption decarbonization to separate a desorbed gas and a decarbonized gas; subjecting the desorbed gas to liquefaction and rectification to obtain liquid carbon dioxide; returning a portion of the decarbonized gas to the blast furnace, and mixing another portion of the decarbonized gas with purified converter gas and coke oven gas subjected to light hydrocarbon conversion, followed by low-temperature methanol wash, temperature swing adsorption, and two-stage pressure swing adsorption to separate CO+N₂ and H₂ streams, which are used for ethylene glycol synthesis. The invention leverages the concept and advantages of steel-coke integration and steel-chemical co-production, converting "carbon" from fuel into raw material, and from emissions into products, thereby achieving a green, low-carbon, and high-efficiency development path. The process primarily utilizes the self-circulation of decarbonized oxygen blast furnace gas to reduce carbon emissions in ironmaking, and uses surplus decarbonized gas, coke oven gas, and converter gas as raw materials for chemical product manufacturing, thereby achieving "carbon fixation and emission reduction" while enhancing value and efficiency, and significantly reducing primary energy consumption from the source.

## Description

### Technical Field

The present invention relates to the field of steelmaking technology, and more particularly, to a near-zero carbon emission integrated process and system for long-process blast furnace steelmaking and chemical co-production.

### Background

The steel industry is a fundamental sector for national economic development. As the center of global steel production and consumption, China currently has the largest steel output in the world, with crude steel production accounting for more than half of the global total, and also has the highest carbon dioxide emissions in the steel sector. Currently, 90% of China's steel industry uses the blast furnace-converter (BF-BOF) long-process production route. Therefore, for the foreseeable future, China will continue to rely on the BF-BOF process, and most steel enterprises are relatively new, making rapid phase-out difficult.

Furthermore, China's energy structure in the steel industry differs from that of other countries. The main fossil energy source is coal, and the proportion of short-process routes such as vertical furnace + electric furnace is relatively low. Under these conditions, low-carbon transformation in the steel industry through energy transition-such as switching from carbon to hydrogen or other energy sources-is technically challenging and costly. Hence, the main technical directions for low-carbon transformation in the steel industry focus on improving the utilization efficiency of existing gas resources, optimizing the energy structure, and supporting CCUS (carbon capture, utilization, and storage).

Currently, in comprehensive utilization of steel enterprise gas, aside from high-value-added special processes such as hydrogen production from coke oven gas or LNG, other utilization methods-such as using blast furnace gas, converter gas, or a mixture of blast furnace and converter gas with coke oven gas as fuel, or for combined-cycle power generation (CCPP)-have relatively low added value. To enhance the comprehensive utilization value of steel enterprise gas, researchers suggest making full use of the chemical energy of the gas, returning part of the gas as a reducing agent to the blast furnace to reduce coke ratio and pulverized coal injection, which not only lowers operational costs of the blast furnace but also improves fossil energy utilization efficiency. Surplus gas can be used as raw material for chemical production, forming a fine processing upstream-downstream chain that substitutes chemical products currently produced from coal. This approach addresses raw material shortages in coal chemistry and reduces equipment cost requirements, while achieving recycling of steel enterprise gas, thereby significantly reducing total fossil fuel consumption and carbon emissions from the source, in line with national carbon reduction policies.

Methanol is a fundamental raw material for a variety of organic products and an important solvent. Upstream raw materials for methanol production mainly include coal, coke oven gas, and natural gas; downstream, methanol is widely used in energy and chemical products. China consumes approximately 70-80 million tons of methanol annually. Currently, coal-based methanol production dominates, accounting for 76% of total production, while coke oven gas-based methanol and natural gas-based methanol account for 17% and 7%, respectively.

Ethylene glycol (EG) is an important organic chemical raw material widely used in antifreeze, polyester fibers (polyurethane, polyethylene terephthalate), nanomaterials, and other chemical products. The domestic and international markets are broad. Although China's ethylene glycol production capacity has been growing at an average annual rate of about 10% in recent years, domestic production still cannot meet growing market demand, with an import dependency rate exceeding 60%. Due to limited petroleum resources, petroleum-derived ethylene glycol has certain limitations. Therefore, producing ethylene glycol via coal-based routes using China's abundant coal resources aligns with the national energy structure and industrial development strategy.

Analysis of coal-based methanol and ethylene glycol production shows that the essence of the coal route is the use of syngas for methanol and ethylene glycol production. Energy consumption in the coal gasification section accounts for over 30% of total energy consumption in coal-based methanol and ethylene glycol production. If syngas is produced without coal gasification, and CO₂ hydrogenation is used to produce methanol and catalytic coupling hydrogenation technology is applied for ethylene glycol synthesis, costs associated with gasification equipment and facilities can be saved, effectively reducing methanol and ethylene glycol production costs and enabling full use of renewable energy. In steel enterprises, coke oven gas contains about 60% H₂, converter gas contains about 60% CO, and oxygen-blown blast furnace gas contains about 45% CO. After purification, these gases can form syngas for methanol and ethylene glycol production, fully utilizing the chemical energy of steel enterprise gas resources.

Based on the above, high-value-added comprehensive utilization of blast furnace, converter, and coke oven gas to reduce steel production costs has always been a key concern for steel enterprises. How to efficiently use gas resources while substantially reducing carbon emissions from steel production remains a challenge to be addressed.

### Summary of the Invention:

To address the low utilization efficiency of existing gas in steel enterprises, the present invention provides a near-zero carbon emission integrated process and system for long-process blast furnace steelmaking and chemical co-production. First, it solves the problem of high energy consumption and low recovery rate in producing decarbonized gas from blast furnace gas; second, it addresses the problem of low purity of captured carbon dioxide products, which is unfavorable for transportation; third, it overcomes the low utilization efficiency of blast furnace gas, converter gas, and coke oven gas, which increases carbon emissions; fourth, it provides a lower-cost pathway for the production of chemical products such as ethylene glycol and methanol.

To achieve the above objectives and other related objectives, a first aspect of the present invention provides a near-zero carbon emission integrated process for long-process blast furnace steelmaking and chemical co-production, comprising the following steps:
subjecting blast furnace gas to pressure swing adsorption decarbonization to separate a desorbed gas and a decarbonized gas;
subjecting the desorbed gas to liquefaction and rectification, thereby obtaining liquid carbon dioxide (liquid CO₂);
returning a portion of the decarbonized gas to the blast furnace, and mixing another portion of the decarbonized gas with purified converter gas and coke oven gas subjected to light hydrocarbon conversion to form a mixed gas;
subjecting the mixed gas stream to low-temperature methanol wash, temperature swing adsorption, and two-stage pressure swing adsorption, thereby separating two streams for ethylene glycol synthesis, wherein one stream comprises carbon monoxide (CO) and nitrogen (N₂), and the other stream comprises hydrogen (H₂).

Further, the liquid carbon dioxide is used for enhanced oil recovery, sequestration, or as a raw material for methanol synthesis;

Further, at least 80% of the decarbonized gas is recycled to the blast furnace, and not more than 20% of the decarbonized gas is mixed with the purified converter gas and the coke oven gas subjected to light hydrocarbon conversion to form the mixed gas.

Further, non-condensable gas separated during the rectification of the desorbed gas is used for cold energy recovery and then returned together with the blast furnace gas for pressure swing adsorption decarbonization to recover reductive components again and achieving 100% recovery of reductive components in the blast furnace gas.

Further, the decarbonized gas and the purified converter gas are subjected to catalytic desulfurization and deoxidation purification, and then mixed with the coke oven gas subjected to light hydrocarbon conversion to form the mixed gas.

Further, the process further comprises the following steps:
subjecting the pretreated blast furnace gas to pressure swing adsorption decarbonization,
subjecting the converter gas produced as a by-product in the converter steelmaking process to pre-treatment to obtain the purified converter gas; and
subjecting the coke oven gas after light hydrocarbon conversion to pretreatment, and then mixing with the decarbonized gas and the purified converter gas to form the mixed gas;
wherein the pre-treatment process comprises at least one procedure selected from dedusting, cooling, desulfurization, and pressurization.

Further, before the low-temperature methanol wash, in the mixed gas, the decarbonized gas, the purified converter gas, and the coke oven gas after light hydrocarbon conversion meet at least one of the following conditions (a) to (d):
(a) the temperature is less than or equal to 40°C;
(b) the oxygen content is less than or equal to 50 ppm;
(c) the hydrogen sulfide content is less than or equal to 0.1 ppm;
(d) the pressure is 2.0-3.5 MPa.

Further, after the low-temperature methanol wash, the mixed gas comprises H₂, CO, N₂, and other impurities, wherein the impurities and their concentration requirements comprise: methanol ≤ 20 ppm, CO₂ ≤ 20 ppm, sulfides ≤ 0.1 ppm, oxygen ≤ 50 ppm.

Further, during the low-temperature methanol wash, the methanol absorbing impurities is subjected to methanol recovery treatment and then returned for recycling in the low-temperature methanol wash, while the separated hydrogen sulfide and CO₂ are introduced into the drying step of the desorbed gas for drying treatment, and subsequently subjected together with the desorbed gas to cryogenic liquefaction and rectification.

Further, after the low-temperature methanol wash, the mixed gas is subjected to temperature swing adsorption to remove heavy impurity components, wherein the heavy impurity components comprise methanol and moisture.

Further, after the temperature swing adsorption, the mixed gas is subjected to two-stage pressure swing adsorption to separate two streams, wherein the two-stage pressure swing adsorption comprises successively performed I-stage pressure swing adsorption and II-stage pressure swing adsorption, the stream separated by the I-stage pressure swing adsorption comprises CO and N₂, and the stream separated by the II-stage pressure swing adsorption comprises H₂.

Further, the synthesis of ethylene glycol adopts catalytic coupling hydrogenation technology, comprising the following steps:
using methyl nitrite as a raw material, and using the stream comprising CO and N₂ as a reactant gas and a diluent to synthesize dimethyl oxalate;
using the dimethyl oxalate synthesized in the preceding step, and using the stream comprising H₂ as a reactant gas to synthesize ethylene glycol.

Further, the process further comprises the step of supplementing hydrogen during the synthesis of ethylene glycol; preferably, the volumetric flow ratio of the stream comprising H2 to the supplemental hydrogen is 1:0.5-1.5; More preferably, the supplemental hydrogen is derived from "green hydrogen" produced from renewable energy, and the oxygen co-produced during the production of green hydrogen from renewable energy is purified and pressurized before being fed into the oxygen blast furnace and converter.

A second aspect of the present invention provides a near-zero carbon emission integrated system for long-process blast furnace steelmaking and chemical co-production, comprising:
a first unit, the first unit comprising a pressure swing adsorption decarbonization system for separating blast furnace gas into a desorbed gas and a decarbonized gas, and a liquefaction and rectification purification system for subjecting the desorbed gas to liquefaction and rectification purification to obtain liquid carbon dioxide;
a second unit, the second unit comprising a catalytic desulfurization and deoxidation system for subjecting the decarbonized gas and purified converter gas to catalytic desulfurization and deoxidation purification;
a third unit, the third unit comprising a light hydrocarbon conversion system for subjecting coke oven gas to light hydrocarbon conversion;
a fourth unit, the fourth unit comprising, arranged in sequence according to the process flow, a gas mixing system, a low-temperature methanol wash system, a temperature swing adsorption system, and a two-stage pressure swing adsorption system, wherein the gas mixing system is connected with the pressure swing adsorption decarbonization system and the light hydrocarbon conversion system, and is used for mixing the decarbonized gas, the purified converter gas, and the coke oven gas after light hydrocarbon conversion to form a mixed gas, and wherein the low-temperature methanol wash system, the temperature swing adsorption system, and the two-stage pressure swing adsorption system are sequentially used for subjecting the mixed gas to low-temperature methanol wash, temperature swing adsorption, and two-stage pressure swing adsorption to separate two streams for synthesizing ethylene glycol, wherein one stream comprises CO and N₂, and the other stream comprises H₂.

Further, the liquefaction and rectification purification system comprises a liquid carbon dioxide outlet, and the system further comprises a methanol synthesis unit connected with the liquid carbon dioxide outlet.

Further, the pressure swing adsorption decarbonization system comprises a decarbonized gas outlet, and the decarbonized gas outlet is connected with a blast furnace reducing gas inlet.

Further, the liquefaction and rectification purification system comprises rectification equipment and a precooler, wherein the rectification equipment comprises a non-condensable gas outlet connected with the precooler, the precooler uses the non-condensable gas as a cold source, and the precooler comprises a cold source outlet connected with an inlet of the pressure swing adsorption decarbonization system.

Further, the system further comprises a first pre-treatment system for pretreating blast furnace gas discharged from the blast furnace;

Further, the system further comprises a second pre-treatment system for pretreating converter gas produced as a by-product in the converter steelmaking process to obtain the purified converter gas.

Further, the system further comprises a third pre-treatment system arranged after a light hydrocarbon conversion system and used for pretreating the coke oven gas after light hydrocarbon conversion.

Further, the first pre-treatment system, the second pre-treatment system, or the third pretreatment system comprises at least one of the following devices: dedusting device, cooling device, desulfurization device, or pressurization device.

Further, the gas mixing system comprises pressurization device for pressurizing the mixed gas.

Further, the system further comprises a methanol recovery system for subjecting methanol absorbing impurities during the low-temperature methanol wash to methanol recovery treatment, wherein the liquefaction and rectification purification system comprises drying device for drying the desorbed gas, and the methanol recovery system is connected with the drying device so as to introduce hydrogen sulfide and CO₂ separated by the methanol recovery treatment into the drying device.

Further, the two-stage pressure swing adsorption system comprises, arranged in sequence according to the process flow, an I-stage pressure swing adsorption device and an II-stage pressure swing adsorption device,
wherein the I-stage pressure swing adsorption device is used for subjecting the mixed gas after temperature swing adsorption to I-stage pressure swing adsorption to separate a stream comprising CO and N₂,
and the II-stage pressure swing adsorption device is used for subjecting the mixed gas after the I-stage pressure swing adsorption to II-stage pressure swing adsorption to separate a stream comprising H₂.

Further, the system further comprises a dimethyl oxalate synthesis unit and an ethylene glycol synthesis unit;
wherein the dimethyl oxalate synthesis unit is connected with the two-stage pressure swing adsorption system and is used for synthesizing dimethyl oxalate by using the stream comprising CO and N₂ as a reactant gas,
and the ethylene glycol synthesis unit is connected with the dimethyl oxalate synthesis unit and the two-stage pressure swing adsorption system and is used for synthesizing ethylene glycol by using the dimethyl oxalate synthesized by the dimethyl oxalate synthesis unit as a raw material and using the stream comprising H₂ as a reactant gas.

Further, the system further comprises a hydrogen supplementation unit, the hydrogen supplementation unit being used for supplementing hydrogen during the process of synthesizing ethylene glycol.

As described above, the near-zero carbon emission integrated process and system for long-process blast furnace steelmaking and chemical co-production of the present invention have the following advantageous effects:

The technical solution of the present invention mainly utilizes a pressure swing adsorption decarbonization system, combined with cooling liquefaction and rectification separation to produce high-purity liquid carbon dioxide; a portion of the decarbonized gas is heated and recycled back to the blast furnace to continue participating in the reduction reaction, resulting in relatively low overall energy consumption; the surplus decarbonized gas, purified converter gas, and coke oven gas after light hydrocarbon conversion are mixed and subjected to low-temperature methanol wash, temperature swing adsorption, and two-stage pressure swing adsorption to separate CO+N₂ and H₂ streams for ethylene glycol synthesis;

Furthermore, by supplementing a portion of hydrogen (preferably green hydrogen), the chemical energy of all three types of steel plant gas resources can be fully utilized, rather than being used in low-efficiency thermal applications.The present invention uses the surplus decarbonized gas, coke oven gas, and converter gas as raw materials for chemical product manufacturing, achieving "carbon fixation and emission reduction" while increasing value and efficiency, thereby significantly reducing primary energy consumption at the source.

Through research and analysis of coal-based methanol and ethylene glycol processes, it has been found that the core of the coal-based route lies in using syngas to produce methanol and ethylene glycol. Firstly, the energy consumption of the coal gasification section accounts for more than 30% of the overall energy consumption. By using a syngas source without undergoing the coal gasification process, combined with the CO₂ hydrogenation process for methanol production and catalytic coupling hydrogenation technology for ethylene glycol production, the construction costs of the coal gasification process and equipment such as gasifiers can be saved, while effectively reducing the production costs of methanol and ethylene glycol and fully utilizing renewable energy.

Secondly, it was found that in the dimethyl oxalate synthesis section of the syngas-based ethylene glycol production process, in addition to adding the reactant gas CO, N₂ must also be introduced from an air separation unit. N₂ functions to absorb the heat released during dimethyl oxalate synthesis and dilute the toxicity of gaseous methyl nitrite; however, the introduction of N₂ increases the gas usage costs in the process. In the present invention, the converter gas, blast furnace gas, and coke oven gas themselves contain approximately 10% N₂, which is not separated from CO during gas separation. This operation not only avoids the costs associated with separating CO and N₂ during purification but also reduces the gas usage costs of reintroducing N₂ during dimethyl oxalate synthesis, thereby effectively lowering ethylene glycol production costs.

In summary, the technical solution provided by the present invention is based on China's national conditions and the actual situation of the steel industry, fully leveraging the unique process advantages of the steel industry. Based on the concept and advantages of steel-coke integration and steel-chemical co-production, it converts "carbon" from fuel into raw material and from emissions into products, following a green, low-carbon, and efficient development path. This solution mainly reduces carbon emissions from blast furnace ironmaking through an oxygen blast furnace gas decarbonization self-circulation system, while utilizing surplus blast furnace gas, coke oven gas, and converter gas as raw materials for chemical products, thereby achieving "carbon fixation and emission reduction" and value-added efficiency, and significantly reducing primary energy consumption at the source.

### Brief Description of the Drawings:

Figure 1 illustrates the diagram of a near-zero carbon emission integrated process for long-process blast furnace steelmaking and chemical co-production according to one embodiment of the present invention.
Figure 2 illustrates the utilization of liquid carbon dioxide in one embodiment of the present invention.
Figure 3 illustrates the process for obtaining liquid carbon dioxide by liquefaction and rectification purification of desorbed gas in one embodiment of the present invention, as well as the layout of the liquefaction and rectification purification system.
Figure 4 illustrates the near-zero carbon emission long-process blast furnace steel-chemical co-production process according to another embodiment of the present invention.
Figure 5 illustrates the near-zero carbon emission integrated process and the layout of the process system according to another embodiment of the present invention.
Figure 6 illustrates the synthesis process of dimethyl oxalate and ethylene glycol, and the layout of the dimethyl oxalate synthesis unit and ethylene glycol synthesis unit according to one embodiment of the present invention.
Figure 7 illustrates the ear-zero carbon emission integrated process system for long-process blast furnace steelmaking and chemical co-production according to another embodiment of the present invention.
Figure 8 illustrates the near-zero carbon emission integrated process and the layout of the process system according to another embodiment of the present invention.

### Detailed Description of the Preferred Embodiments:

The embodiments of the present invention are described below with reference to specific examples, through which those skilled in the art can readily understand other advantages and effects of the present invention as disclosed in this specification. The present invention may also be implemented or applied through other different specific embodiments, and the various details described in this specification may be modified or altered from different perspectives and applications without departing from the spirit of the present invention.

It should be noted that the illustrations provided in this embodiment are intended solely to schematically illustrate the basic concept of the present invention. Accordingly, the drawings show only components relevant to the present invention, rather than depicting the actual number, shape, or dimensions of components in practical implementations. In actual embodiments, the forms, quantities, and proportions of the components may be arbitrarily varied, and the layout of the components may be more complex. The structures, proportions, and sizes shown in the drawings of this specification are provided solely to assist in understanding and reading the disclosed content and are not intended to limit the scope of the present invention. Therefore, any modifications to the structure, changes in proportional relationships, or adjustments of sizes, so long as they do not affect the effects achievable by the present invention or the objectives to be accomplished, should still fall within the scope encompassed by the technical content disclosed herein.

Moreover, terms used in this specification such as "upper," "lower," "left," "right," "middle," and "one" are employed merely for convenience of description and clarity, and are not intended to limit the scope of the present invention. Any changes or adjustments in the relative relationships of these terms, without substantial alteration of the technical content, should likewise be considered within the scope of the present invention.

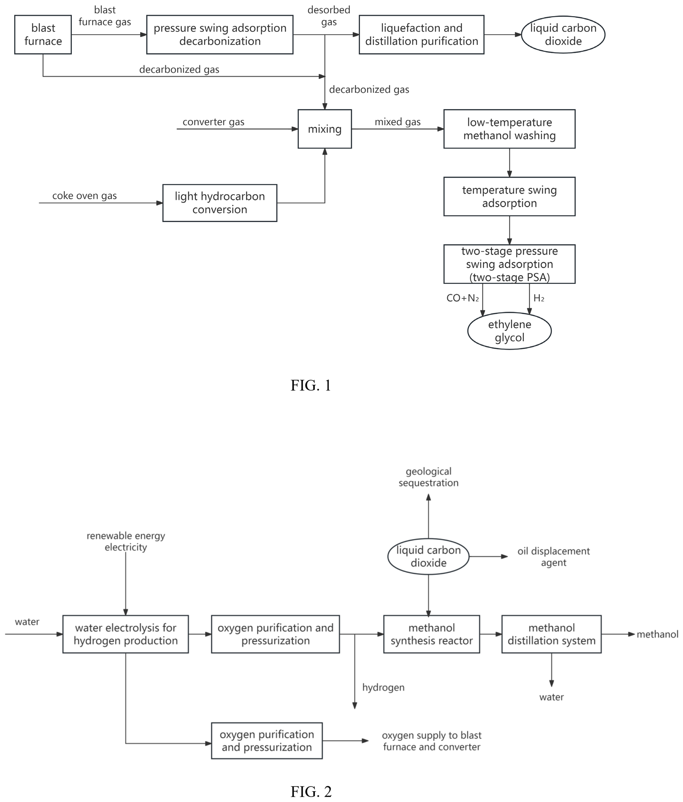

First, it should be noted that in the following embodiments and in Figures 1-8, the abbreviations refer to the following:
MN - Methyl Nitrite, chemical formula: CH₃ONO;
DMO - Dimethyl Oxalate, chemical formula: (COOCH₃)₂;
DMC - Dimethyl Carbonate, chemical formula: CO(OCH₃)₂;
MG - Methyl Glycolate, chemical formula: HOCH₂CO₂CH₃ ;
EG - Ethylene Glycol, chemical formula: HOCH₂CH₂OH;
ET - Ethanol, chemical formula: CH₃CH₂OH;
TSA - Temperature Swing Adsorption;
PSA - Pressure Swing Adsorption.

Referring to Figure 1, Figure 1 is a schematic diagram of the process flow of a near-zero carbon emission integrated process for long-process blast furnace steelmaking and chemical co-production according to one embodiment of the present application.

As shown in Figure 1, the disclosed embodiment provides a near-zero carbon emission integrated process for long-process blast furnace steelmaking and chemical co-production, comprising the following steps:
Subjecting blast furnace gas to pressure swing adsorption decarbonization to separate it into desorption gas and decarbonized gas;
Treating the desorption gas via liquefaction and distillation purification to obtain liquid carbon dioxide;
Returning a portion of the decarbonized gas to the blast furnace, while mixing the remaining portion with refined converter gas and coke oven gas treated via light hydrocarbon conversion to form a mixed gas;
Subjecting the mixed gas to low-temperature methanol wash, temperature swing adsorption, and two-stage pressure swing adsorption to separate it into two streams for ethylene glycol synthesis, wherein one stream comprises CO and N₂, and the other stream comprises H₂.

The blast furnace gas is subjected to pressure swing adsorption decarbonization to separate desorption gas and decarbonized gas using vacuum pressure swing adsorption technology. Specifically, the blast furnace gas first enters the adsorption tower (multi-tower configuration) from the bottom. After CO₂ components are adsorbed by the adsorbent in the tower, the decarbonized gas is discharged from the top of the adsorption tower. The adsorbed components in the adsorption tower constitute the desorption gas. Initially, a portion of the desorption gas is released by reducing the pressure. When the pressure in the adsorption tower drops close to atmospheric pressure, a vacuum pump is used to extract the remaining desorption gas completely, which is then discharged into a desorption gas buffer tank.

In some embodiments, the liquid carbon dioxide is used for enhanced oil recovery, sequestration, or as a raw material for methanol synthesis.

As shown in Figure 2, the liquid carbon dioxide can be directly delivered to geological sequestration sites or oil fields for enhanced oil recovery; or, methanol can be synthesized via a carbon dioxide hydrogenation method, for example, by reacting with "green hydrogen" produced from renewable energy. The methanol obtained after rectification and purification can be sold as a product.

The "green hydrogen" produced from renewable energy can be used together with liquid carbon dioxide to synthesize the chemical product methanol. The implementation route is as follows: hydrogen is produced by water electrolysis using renewable energy electricity, and the hydrogen, after purification and pressurization, is sent into a methanol synthesis reactor; the liquid carbon dioxide also enters the reactor and reacts with the hydrogen to produce methanol. The generated methanol is purified via a methanol rectification system and can be sold as a product. Meanwhile, the by-product oxygen generated during water electrolysis for hydrogen production, after purification and pressurization, can be supplied to the oxygen blast furnace and converter. In some embodiments, at least 80% of the decarbonized gas is recycled to the blast furnace, while no more than 20% of the decarbonized gas is mixed with the purified converter gas and the coke oven gas subjected to light hydrocarbon conversion to form a mixed gas.

In some embodiments, the decarbonized gas is heated and recycled to the blast furnace as a reducing gas, preferably heated to 800-1000°C. By heating the decarbonized gas to 800-1000°C and circulating it to the blast furnace as a reducing agent, not only is the blast furnace gas recycled and the content of reducing gas in the bosh gas increased, but indirect reduction in the blast furnace is promoted, the proportion of direct reduction is reduced, thereby decreasing the consumption of coke and coal fuel during the blast furnace ironmaking process, and effectively reducing CO₂ emissions.

In embodiments of the present application, blast furnace gas refers to the gas generated during ironmaking in an oxygen blast furnace to produce molten iron. Since the blast furnace gas generated during oxygen blast furnace ironmaking is characterized by high dust content, high sulfur content, high temperature, and low pressure, pre-treatment of the blast furnace gas is required prior to pressure swing adsorption decarbonization.

In some embodiments, the pre-treatment process of the blast furnace gas includes at least one of the following steps: dedusting, cooling, and desulfurization. Preferably, the pretreated blast furnace gas has the following characteristics: dust content less than 10 mg/Nm³, temperature reduced to 30-40°C, H₂S content less than 10 mg/Nm³, and pressurized to 0.2-0.8 MPa.

In some embodiments, the decarbonized gas obtained by pressure swing adsorption decarbonization contains less than 1% CO₂, and the pressure is 0.60-0.75 MPa.

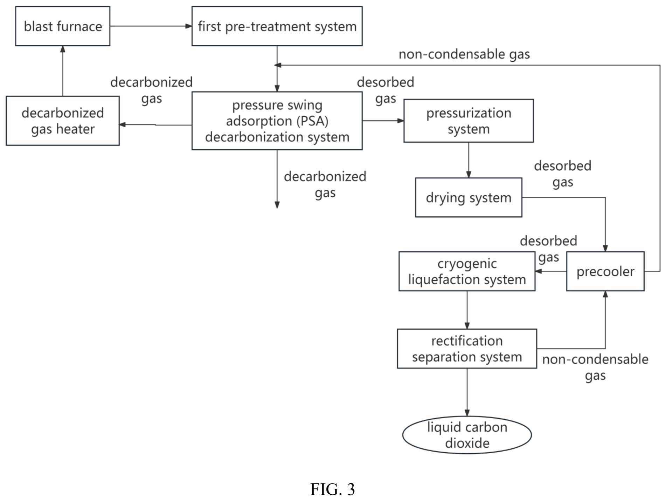

In some embodiments, as shown in Figure 3, the desorbed gas is subjected to liquefaction and rectification purification to obtain liquid carbon dioxide. The treatment process comprises the following steps: pressurization, drying, cryogenic liquefaction, and rectification separation. The non-condensable gas separated during rectification (mainly comprising CO and CO₂) is returned after cold energy recovery, and together with the blast furnace gas, subjected again to pressure swing adsorption decarbonization, thereby further recovering the reductive components and achieving 100% recovery of the reductive components in the blast furnace gas.

In some embodiments, when the desorbed gas is pressurized, the compressor outlet pressure of the desorbed gas pressurization system is 2.50-3.0 MPa, the outlet temperature is 30-40°C, and no impurities are added to the outlet medium, with the free water content not exceeding 7 g/Nm³.

The pressurized desorbed gas first enters a drying system for drying treatment, which includes a dryer. The purpose of drying is to remove moisture from the desorbed gas to prevent freezing during subsequent cryogenic liquefaction, which could cause pipeline or equipment blockage. The dried desorbed gas then enters a precooler, where the non-condensable gas from the top of the rectification tower is used as a cold source to reduce the temperature to 10-30°C. Subsequently, the precooled desorbed gas enters a cryogenic liquefaction system for cryogenic liquefaction treatment. The cryogenic liquefaction system comprises a refrigerant to lower the gas temperature to -30--40°C for liquefaction. The liquefied desorbed gas then enters a rectification separation system for rectification purification, which includes a rectification tower. During rectification, high-purity liquid carbon dioxide is discharged from the bottom of the rectification tower, and the non-condensable gas discharged from the top, after cold energy recovery, is returned to the inlet of the adsorption tower and subjected again to pressure swing adsorption decarbonization together with blast furnace gas, thereby achieving recovery of the reductive components.

In some embodiments, the purified converter gas refers to the converter gas produced as a by-product in the converter steelmaking process, which is obtained after pre-treatment and pressurization. The pre-treatment of converter gas includes dedusting, cooling, and desulfurization.

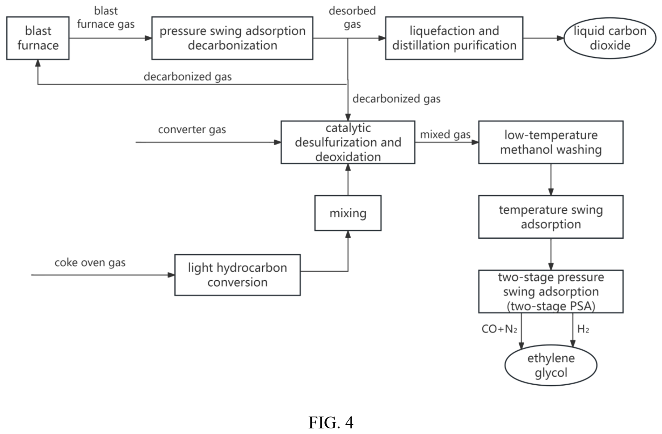

In some embodiments, as shown in Figure 4, the decarbonized gas and the purified converter gas are subjected to catalytic desulfurization and deoxidation purification, and then mixed with the coke oven gas after light hydrocarbon conversion to form a mixed gas.

Since the converter gas and surplus decarbonized gas contain a certain amount of sulfides, mainly COS and H₂S, and COS must first be converted to H₂S before removal, and since the converter gas and decarbonized gas streams contain H₂, a reduction method is used for COS conversion, namely hydrogenation conversion. In some embodiments, the reaction conditions for hydrogenation conversion are 200-250°C and 0.5-1.0 MPa, using a Ni-Mo based catalyst with γ-Al₂O₃ as the carrier. After hydrogenation conversion, the volume content of COS in the gas can be reduced to 4×10⁻⁸.

Moreover, the mixed gas formed by mixing the converter gas and the surplus decarbonized gas contains approximately 0.30% oxygen. That is, the mixed gas in this unit contains a small amount of O₂ and sufficient H₂, which can be used for deoxidation. Therefore, in some embodiments, the reaction conditions for catalytic deoxidation are 60-150°C and 0.5-2.0 MPa, using a Pt-γ-Al₂O₃ catalyst. Under the action of the catalyst, the O₂ in the gas reacts with H₂ to generate H₂O, reducing the oxygen content in the mixed gas to below 150 ppm.

In some embodiments, the coke oven gas after light hydrocarbon conversion refers to the high-temperature coke oven gas produced as a by-product during coking in a coke oven, which is subjected to non-catalytic conversion to perform light hydrocarbon conversion. The high-temperature coke oven gas generated during coking has a temperature of 700-800°C. To fully utilize its sensible heat and avoid a purification process, non-catalytic partial oxidation technology is employed. The reaction temperature of non-catalytic partial oxidation is 1200-1500°C, and the reactants include high-temperature coke oven gas, high-pressure steam at 350-400°C, and oxygen at 200-300°C. No catalyst is placed in the non-catalytic conversion furnace. After the reaction, light hydrocarbons and compounds such as benzene in the gas are completely consumed, generating high-temperature, high-pressure gas. The reaction heat is recovered through a multi-stage heat exchange system, mainly used for raw material preheating and steam production via heat absorption by desalted water. Subsequently, the high-temperature gas is cooled through multi-stage heat exchange and water is removed via a separation tank. The dehydrated gas is then purified, dedusted, and pressurized before being sent to the low-temperature methanol wash process.

In some embodiments, the coke oven gas after light hydrocarbon conversion, after pretreatment, is mixed with the decarbonized gas and the purified converter gas to form mixed gas. The pre-treatment of the coke oven gas after light hydrocarbon conversion includes dedusting and multi-stage heat exchange cooling.

In the above embodiments, the dedusting, cooling, desulfurization, and pressurization of the blast furnace gas, converter gas, and coke oven gas can be carried out through the following methods:
Dedusting: performed by gravity dedusting, bag filter dedusting, or a combination of both.
Cooling: the gas is cooled through a heat exchanger.
Desulfurization: sulfides in the gas are removed by physical adsorption, chemical desulfurization, or a combination thereof.
Pressurization: performed by a compressor, which may be a screw compressor or a centrifugal compressor, with an outlet pressure of 0.65-0.80 MPa, outlet temperature of 30-40°C, no impurities added to the outlet medium, and free water content not exceeding 7 g/Nm³.

In some embodiments, before low-temperature methanol washing, the decarbonized gas, purified converter gas, and coke oven gas after light hydrocarbon conversion in the mixed gas must satisfy at least one of the following conditions (a) to (d):
(a) Temperature is less than or equal to 40°C;
(b) Oxygen content is less than or equal to 50 ppm;
(c) Hydrogen sulfide content is less than or equal to 0.1 ppm;
(d) Pressure is 2.0-3.5 MPa.

In some embodiments, the mixed gas after low-temperature methanol washing mainly comprises H₂, CO, N₂, and other impurities, wherein the other impurities and their concentration limits are as follows: methanol ≤20 ppm, CO₂ ≤20 ppm, sulfides ≤0.1 ppm, oxygen ≤50 ppm.

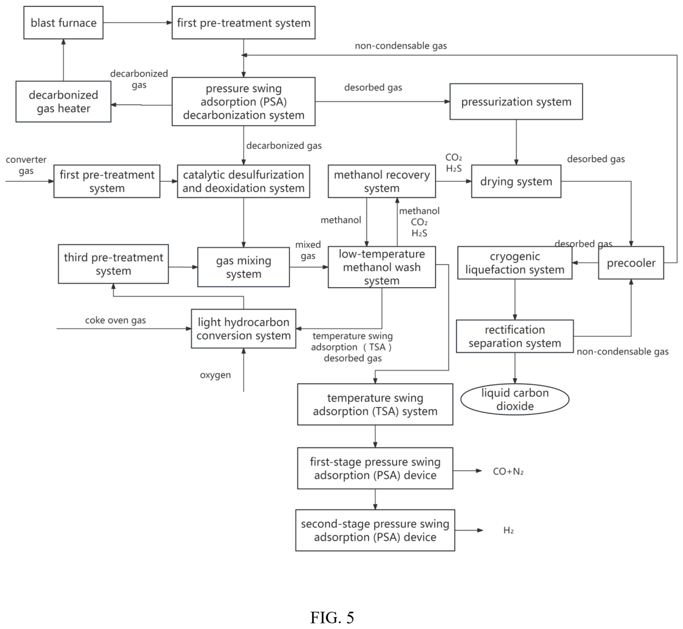

In some embodiments, as shown in Figure 5, the methanol that absorbs impurities during the low-temperature methanol washing process is recovered and returned for circulation. The separated impurities, hydrogen sulfide and CO₂, are sent to the drying process of the desorbed gas, and then undergo cryogenic liquefaction and rectification together with the desorbed gas.

In the above embodiments, the purified converter gas and surplus decarbonized gas are mixed with the coke oven gas that has undergone non-catalytic conversion, purification, and pressurization, and then together enter the low-temperature methanol wash system. In the low-temperature methanol wash system, the mixed gas comes into countercurrent contact with low-temperature methanol at -55 to -60°C. The methanol absorbs impurities such as hydrogen sulfide and CO₂ from the gas, thereby purifying it. After processing through absorption, flashing, desorption, and thermal regeneration units, the main components of the gas are H₂, CO, and N₂, while the concentrations of other impurities are reduced to meet the purity requirements: methanol ≤20 ppm, CO₂ ≤20 ppm, sulfides ≤0.1 ppm, oxygen ≤50 ppm. The methanol that absorbs impurities is treated by a methanol recovery system and returned for circulation in the low-temperature methanol wash process. The separated hydrogen sulfide and CO₂ are sent to the drying system of the desorbed gas after blast furnace gas decarbonization, and then together with the decarbonized desorbed gas from the blast furnace enter the liquefaction and rectification purification system.

In some embodiments, the mixed gas after low-temperature methanol washing first undergoes a temperature-swing adsorption (TSA) process to remove heavy impurity components, wherein the heavy impurity components include methanol and water. Further, the adsorption beds are regenerated using heated hydrogen, enabling continuous operation of the temperature swing adsorption process.

In some embodiments, the mixed gas treated by the temperature swing adsorption process subsequently undergoes a two-stage pressure swing adsorption (PSA) process to separate two gas streams. The two-stage PSA comprises a first-stage PSA (Stage I) and a second-stage PSA (Stage II) carried out sequentially:

The gas stream separated by Stage I PSA includes CO and N₂, preferably with H₂ content ≤50 ppm;

The gas stream separated by Stage II PSA mainly consists of H₂, preferably with CO content ≤20 ppm.

The mixed gas treated by the temperature swing adsorption enters the two-stage PSA system. The Stage I PSA unit mainly separates the CO and N₂ components. The H₂ content in the CO+N₂ stream must be strictly controlled because N₂ is toxic to the DMO synthesis catalyst. After pressurization to 0.5 MPa, this stream is sent to the dimethyl oxalate (DMO) synthesis unit. The hydrogen component discharged from the top of the adsorption tower in the Stage I PSA unit is sent to the Stage II PSA unit for further processing. In this process, the CO content in the H₂ product must be strictly controlled, as the gas feed for the ethylene glycol (EG) synthesis unit must be free of CO. The H₂ discharged from the tower top is then used as the hydrogen feed for the hydrogenation of DMO to produce EG, and is sent to the ethylene glycol synthesis reactor.

In some embodiments, the ethylene glycol synthesis process employs catalytic coupling hydrogenation technology, comprising the following steps:
using methyl nitrite (MN) as a feedstock, and the gas stream containing CO and N₂ as feed gas and diluent, to synthesize dimethyl oxalate (DMO);
using the DMO synthesized in step 1 as feedstock, and the gas stream containing H₂ as feed gas, to synthesize ethylene glycol.

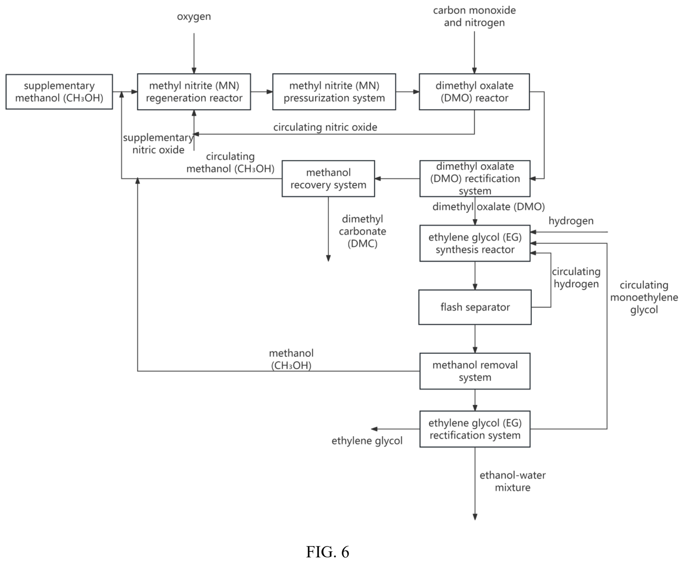

As shown in Figure 6, in some embodiments, the DMO and ethylene glycol synthesis process is as follows:

CO separated from the mixed gas enters the DMO synthesis unit, which comprises a DMO reactor, a DMO distillation system, and an MN pressurization system. To improve raw material utilization, the unit may further include an MN regeneration reactor and a methanol recovery system. The DMO synthesis process in the unit can be divided into two reaction steps:

NO, O₂, and CH₃OH react during the regeneration process to generate methyl nitrite (MN), after which CO reacts with MN under catalytic conditions to undergo a coupling reaction to synthesize DMO. The chemical reactions and specific reaction process involved are as follows:

4CH₃OH+O₂+4NO=4CH₃ONO+2H₂O

2CO+2CH₃ONO=(COOCH₃)₂+2NO (main reaction)

CO+2CH₃ONO=CO(OCH₃)₂+2NO(side reaction)

The MN regeneration reaction process is as follows:
NO and O₂ are mixed and introduced from the bottom of the MN regeneration reactor distillation tower; CH₃OH is sprayed from the top of the distillation tower. The gas at the bottom and the liquid at the top contact each other countercurrently to react at a temperature of 25-150°C and a pressure of 0.5 MPa, generating MN in the gas phase. The reaction products are then separated by the distillation and rectification sections of the MN regeneration reactor distillation tower.

The top gas (NO, MN, O₂) is pressurized and then applied to the liquid flowing from the bottom of the tower (water and methanol). The liquid enters the ordinary distillation column of the MN regeneration reactor for separation of methanol and water. Methanol recovered from the tower top is returned to the top of the MN regeneration reactor distillation column for recirculation.

The top gas (NO, MN, O₂), after being pressurized by the MN pressurization system, is mixed with the pressurized CO and N₂ and fed into the DMO synthesis reactor. In this process, CO serves as the reaction feedstock, and N₂ absorbs the heat released during DMO synthesis and dilutes the toxicity of MN, because the catalytic coupling reaction between CO and MN is strongly exothermic, and MN is a toxic gas.

The product from the DMO reactor is a gas-liquid mixture, which first enters a flash separation tank for gas-liquid separation. The liquid phase mainly consists of DMO, dimethyl carbonate (DMC), and CH₃OH, and is sent to the DMO distillation system for purification. The top stream of the distillation column is DMC/CH₃OH, while the bottom stream is DMO. The purified DMO is then sent to the ethylene glycol (EG) synthesis unit for hydrogenation with H₂ to produce EG.

The gas phase, mainly consisting of CO, N₂, NO, etc., is supplemented with O₂ and returned to the bottom of the MN regeneration reactor distillation column to participate in the MN regeneration reaction.

After obtaining high-purity DMO through the CO coupling reaction, DMO enters the EG synthesis unit. In this unit, DMO reacts with H₂ in a hydrogenation reaction to form EG. The reaction products subsequently undergo heat exchange, distillation separation, and other processing steps to obtain the target product, ethylene glycol. The EG synthesis unit comprises an EG synthesis reactor, flash separator, methanol removal system, and EG distillation system. The process mainly includes two reaction steps and one separation step:
First reaction step: DMO reacts withH₂ to form methyl glycolate (MG)

   DMO+2H₂=MG+CH₃OH
Second reaction step: MG further reacts with H₂ to produce EG, with a side reaction generating ethanol (ET)

   MG+2H₂=EG+CH₃OH (main reaction)

   EG+H₂=ET+H₂O (side reaction)

The DMO feedstock from the DMO synthesis unit is mixed with H₂ and, after heat exchange with the EG synthesis reactor effluent, enters the EG synthesis reactor. The reactor effluent undergoes heat exchange with the feed gas and is then cooled to a suitable temperature before entering the flash separation tank for gas-liquid separation. The separated gas phase is recycled and mixed with the DMO feedstock for re-entry into the EG synthesis reactor. The liquid phase passes through the methanol removal system, methanol recovery tower, dehydration column, and EG distillation column to obtain the final EG product.

In some embodiments, the process further comprises the following step: supplementing hydrogen during the synthesis of ethylene glycol, wherein, in a preferred embodiment, the volumetric flow ratio of the H₂ stream to the supplementary hydrogen is 1:0.5~1.5. The hydrogen may be sourced from "green hydrogen" produced using renewable energy.

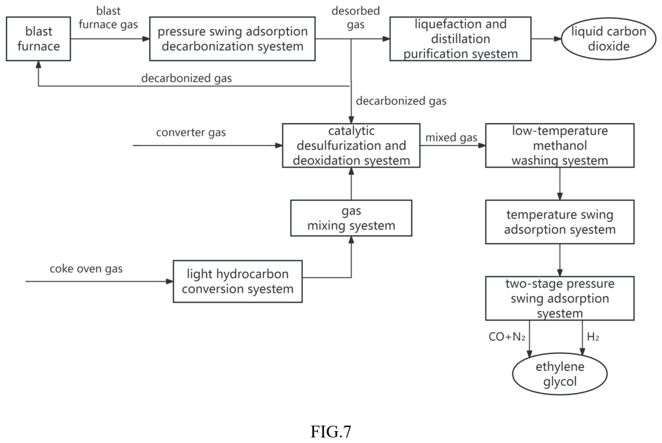

Referring to Figure 7, Figure 7 illustrates a schematic layout of a near-zero carbon emission integrated process system for long-process blast furnace steelmaking and chemical co-production according to another embodiment of the present application.

As shown in Figure 7, another disclosed embodiment of the present application provides a near-zero carbon emission integrated process system for long-process blast furnace steelmaking and chemical co-production, comprising:
First unit, the first unit comprising a pressure swing adsorption decarbonization system for separating blast furnace gas into desorbed gas and decarbonized gas, and a liquefaction and rectification purification system for obtaining liquid carbon dioxide from the desorbed gas.
Second unit, the second unit comprising a catalytic desulfurization and deoxidation system for catalytically removing sulfur and oxygen from the decarbonized gas and refined converter gas.
Third unit, the third unit comprising a light hydrocarbon conversion system for converting coke oven gas.
Fourth unit, the fourth unit sequentially comprising a gas mixing system, a low-temperature methanol wash system, a temperature-swing adsorption system, and a two-stage pressure swing adsorption system. The gas mixing system is connected to the pressure swing adsorption decarbonization system and the light hydrocarbon conversion system for mixing the decarbonized gas, refined converter gas, and light hydrocarbon-converted coke oven gas to form a mixed gas. The low-temperature methanol wash system, temperature-swing adsorption system, and two-stage pressure swing adsorption system are sequentially configured to perform low-temperature methanol washing, temperature-swing adsorption, and two-stage pressure swing adsorption on the mixed gas to separate two streams for ethylene glycol synthesis, wherein one stream comprises CO and N₂, and the other stream comprises H₂.

In some embodiments, the liquefaction and rectification purification system includes a liquid carbon dioxide outlet, and the process system further comprises a methanol synthesis unit connected to the liquid carbon dioxide outlet.

In some embodiments, the pressure swing adsorption decarbonization system includes a decarbonized gas outlet, which is connected to the inlet of the blast furnace reducing gas.

In some embodiments, as shown in Figure 3, the liquefaction and rectification purification system comprises a rectification apparatus and a precooler. The rectification apparatus includes a non-condensable gas outlet, which is connected to the precooler. The precooler uses the non-condensable gas as a cooling source and includes a cooling source outlet connected to the inlet of the pressure swing adsorption decarbonization system.

In some embodiments, the first unit further comprises a first pre-treatment system for pretreating the blast furnace gas discharged from the blast furnace.

In some embodiments, the second unit further comprises a second pre-treatment system for pre-treating the converter gas, which is a by-product of the converter steelmaking process, to obtain refined converter gas.

In some embodiments, the third unit further comprises a third pre-treatment system arranged downstream of the light hydrocarbon conversion system for pre-treating the light hydrocarbon-converted coke oven gas.

In some embodiments, the first pre-treatment system, the second pre-treatment system, or the third pre-treatment system comprises at least one of the following devices: dedusting device, cooling device, desulfurization device, or pressurization device.

In some embodiments, the gas mixing system comprises pressurization device for boosting the mixed gas.

In some embodiments, as shown in Figure 5, the fourth unit further comprises a methanol recovery system for recovering methanol that has absorbed impurities during the low-temperature methanol wash; the liquefaction and rectification purification system includes drying device for drying the desorbed gas, and the methanol recovery system is connected to the drying device to deliver the impurities, H₂S and CO₂, separated during methanol recovery to the drying device.

In some embodiments, as shown in Figure 5, the two-stage pressure swing adsorption system comprises, sequentially along the process flow, a first-stage (stage I) pressure swing adsorption device and a second-stage (stage II) pressure swing adsorption device. The first-stage pressure swing adsorption device performs first-stage adsorption on the mixed gas treated by temperature-swing adsorption to separate a stream comprising CO and N₂; the second-stage pressure swing adsorption device performs second-stage adsorption on the mixed gas treated by the first-stage adsorption to separate a stream comprising H₂.

In some embodiments, as shown in Figure 6, the process system further comprises a dimethyl oxalate (DMO) synthesis unit and an ethylene glycol (EG) synthesis unit. The DMO synthesis unit is connected to the two-stage pressure swing adsorption system and is configured to synthesize DMO using the stream comprising CO and N₂ as feed gas. The EG synthesis unit is connected to both the DMO synthesis unit and the two-stage pressure swing adsorption system and is configured to synthesize EG using DMO produced by the DMO synthesis unit as feedstock and the stream comprising H₂ as feed gas.

In some embodiments, the process system further comprises a hydrogen supplementation unit, which is configured to supplement hydrogen during the synthesis of EG.

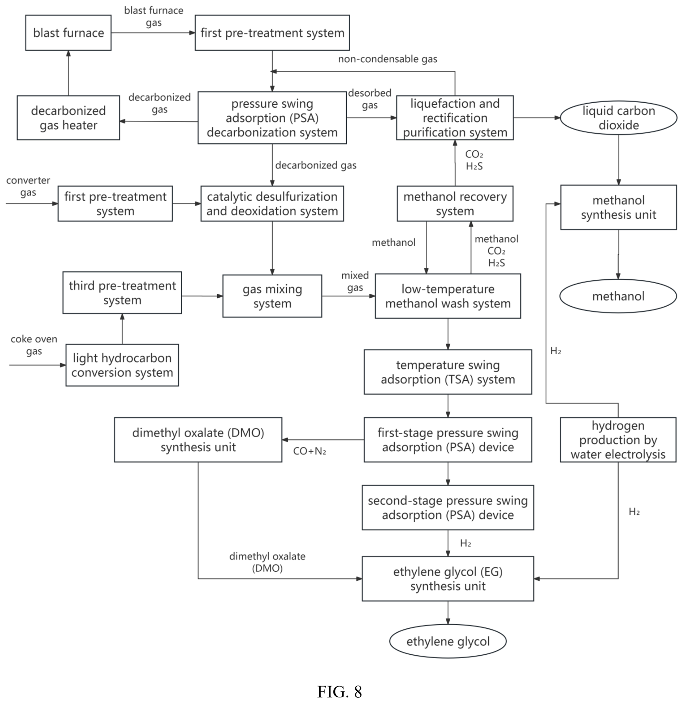

Referring to Figure 8, Figure 8 illustrates a flow diagram and process system layout of a near-zero carbon emission integrated process for long-process blast furnace steelmaking and chemical co-production according to another embodiment of the present application.

As shown in Figure 8, another disclosed embodiment provides a near-zero carbon emission integrated process and system for long-process blast furnace steelmaking and chemical co-production. The specific implementation is as follows:
1. This embodiment is based on an oxygen blast furnace with a volume of 530 m³, with the parameter 1:

**Parameter 1**

| Utilization factor(t/(d·m³)) | Hot metal production(t/h) | Top dry gas volume(m³/t) | Blowing gas volume(m³/t) | Top gas recirculation ratio | Dry coke ratio(kg/t) |
|---|---|---|---|---|---|
| 4.151 | 91.668 | 1126 | 590 | 0.867 | 305 |

1) Raw material 1: blast furnace gas, with volumetric fractions of CO 44.30%, CO₂ 40.23%, H₂ 10.57%, N₂ 4.70%, O₂ 0.20%; pressure 0.15 MPa; temperature 150°C; blast furnace gas flow 103,218 Nm³/h.
2) Raw material 2: coke oven gas, with volumetric fractions of H₂ 60.50%, CH₄ 25%, CO 8.00%, CO₂ 2.50%, N₂ 1.50%, C₂H₄ 1.50%, C₂H₆ 0.80%, O₂ 0.20%; pressure 0.015 MPa; temperature 750°C; coke oven gas flow 11,183 Nm³/h (based on 400 Nm³/t of coke).
3) Raw material 3: converter gas, with volumetric fractions of CO 53.70%, N₂ 26.80%, CO₂ 17.20%, H₂ 2.00%, CH₄ 0.10%, O₂ 0.20%; pressure 0.012 MPa; temperature 40°C; converter gas flow 10,083 Nm³/h (based on 110 Nm³/t of steel).
4) Raw material 4: supplementary green hydrogen, with volumetric fractions of H₂ 99.99%, N₂ 0.01%; flow 13,621 Nm³/h; pressure 2.5 MPa; temperature 40°C.

2. As shown in FIG. 8, the process system of the present embodiment includes:
First unit, the first unit includes a first pre-treatment system, a pressure swing adsorption decarbonization system, a liquefaction and rectification system, and a decarbonized gas heater. The first pre-treatment system comprises, sequentially connected according to the process flow, a dedusting device, a cooling device, a desulfurization device, and a pressurization device. The blast furnace gas pressurized by the pressurization device enters the pressure swing adsorption decarbonization system, which is configured to separate the blast furnace gas into desorbed gas and decarbonized gas. The decarbonized gas separated by the pressure swing adsorption decarbonization system is discharged from a decarbonized gas outlet, and the decarbonized gas outlet is sequentially connected to the decarbonized gas heater and the blast furnace injection system; the desorbed gas separated by the system is discharged from a desorbed gas outlet, and the desorbed gas outlet is connected to the liquefaction and rectification system. The liquefaction and rectification system is configured to liquefy and purify the desorbed gas to obtain liquid carbon dioxide, and comprises, sequentially connected according to the process flow, a pressurization system, a drying system, a deep cryogenic liquefaction system, a rectification separation system, and a precooler. The rectification separation system is provided with rectification equipment, the rectification equipment includes a non-condensable gas outlet, and the outlet is connected to the precooler. The precooler uses the non-condensable gas as a cold source and includes a cold source outlet, which is connected to the inlet of the pressure swing adsorption decarbonization system.
Second unit, the second unit includes a second pre-treatment system and a catalytic desulfurization and deoxygenation system, sequentially connected according to the process flow. The second pre-treatment system is configured to pre-treat the converter gas, a by-product of the steelmaking process in the converter, to obtain refined converter gas, and comprises, sequentially connected according to the process flow, a dedusting device, a cooling device, a desulfurization device, and a pressurization device. The catalytic desulfurization and deoxygenation system is configured to catalytically desulfurize and deoxygenate the decarbonized gas and the refined converter gas, and the decarbonized gas outlet of the pressure swing adsorption decarbonization system is also connected to the inlet of the catalytic desulfurization and deoxygenation system.
Third unit, the third unit includes a light hydrocarbon conversion system and a third pretreatment system. The light hydrocarbon conversion system is configured to convert coke oven gas; the third pre-treatment system is arranged downstream of the light hydrocarbon conversion system and is configured to pre-treat the coke oven gas after light hydrocarbon conversion, comprising a dedusting device, a cooling device, and a desulfurization device.
Fourth unit, the fourth unit includes, sequentially according to the process flow, a gas mixing system, a low-temperature methanol wash system, a temperature swing adsorption system, and a two-stage pressure swing adsorption system. The gas mixing system is connected to the catalytic desulfurization and deoxygenation system and the third pre-treatment system, and is configured to mix the decarbonized gas, the refined converter gas, and the coke oven gas after light hydrocarbon conversion to form mixed gas, and includes pressurization equipment for pressurizing the mixed gas. The low-temperature methanol wash system and the temperature swing adsorption system are sequentially configured to perform low-temperature methanol washing and temperature swing adsorption on the mixed gas. The two-stage pressure swing adsorption system includes a first-stage pressure swing adsorption device and a second-stage pressure swing adsorption device sequentially arranged according to the process flow. The first-stage pressure swing adsorption device is configured to perform first-stage pressure swing adsorption on the mixed gas after temperature swing adsorption, separating a stream comprising CO and N₂; the second-stage pressure swing adsorption device is configured to perform second-stage pressure swing adsorption on the mixed gas after first-stage pressure swing adsorption, separating a stream comprising H₂.

The fourth unit further comprises a methanol recovery system for recovering methanol that has absorbed impurities during the low-temperature methanol wash process, the methanol recovery system being connected to the desorption gas drying system, and configured to deliver the impurities separated through methanol recovery, including hydrogen sulfide and CO₂, into the drying device.

The methanol synthesis unit is connected to the liquid carbon dioxide outlet of the liquefaction and purification system.

The dimethyl oxalate (DMO) synthesis unit is connected to the I-stage pressure swing adsorption (PSA) device, and is configured to synthesize dimethyl oxalate using a gas stream including CO and N₂ as the feed gas. Referring to Figure 6, the DMO synthesis unit comprises, sequentially connected according to the process flow, an MN regeneration reactor, an MN pressurization system, a DMO reactor, and a DMO distillation system.

The ethylene glycol (EG) synthesis unit is connected to the DMO synthesis unit and the II-stage PSA device, and is configured to synthesize ethylene glycol using dimethyl oxalate produced by the DMO synthesis unit as the feedstock and a gas stream including H₂ as the feed gas. Referring to Figure 6, the EG synthesis unit comprises, sequentially connected according to the process flow, an EG synthesis reactor, a flash separator, a methanol removal system, and an EG distillation system.

The hydrogen supplementation unit is configured to supplement hydrogen during the synthesis of ethylene glycol. The hydrogen supplementation unit can be implemented, as shown in Figure 2, by hydrogen production through water electrolysis using renewable electricity.

3. As shown in Figure 1, the process flow of the present embodiment is as follows:
Step 1: The blast furnace gas first undergoes dust removal, desulfurization, cooling, and pressurization, with the temperature reduced to below 40 °C, dust content below 10 mg/Nm³, H₂S removed to 1 ppm, and pressurized to 0.2-0.8 MPa.
Step 2: The pressurized blast furnace gas is introduced into the PSA decarbonization system. Of the high-pressure decarbonized gas discharged from the top of the PSA decarbonization system, 80% is heated by a heater to 800-1000 °C and recycled to the blast furnace, while the remaining 20% of the decarbonized gas is sent to the catalytic desulfurization and deoxygenation system. The desorbed gas discharged from the bottom of the PSA decarbonization system, after depressurization, is mixed with vacuum-discharged desorbed gas and then pressurized to 2.0-3.0 MPa, dried, deep-cooled, liquefied, and distilled, finally obtaining a high-pressure liquid CO₂ product at the bottom of the distillation column. The non-condensable gas recovered from the top of the distillation column returns to the inlet of the PSA decarbonization system after providing cooling.
Step 3: The converter gas first undergoes dust removal, desulfurization, cooling, and pressurization to obtain refined converter gas. The refined converter gas is mixed with surplus decarbonized gas for desulfurization and deoxygenation purification, and finally pressurized and sent to the low-temperature methanol wash system.
Step 4: The high-temperature coke oven gas is directly introduced into the non-catalytic conversion furnace of the light hydrocarbon conversion system for light hydrocarbon conversion, then undergoes dust removal, multi-stage heat exchange cooling, and pressurization to 0.70 MPa. It is then mixed with refined converter gas and surplus decarbonized gas to form a mixed gas, which is further pressurized to 3.0 MPa and sent to the low-temperature methanol wash system.
Step 5: The mixed gas undergoes low-temperature methanol decarbonization, followed by temperature-swing adsorption and a two-stage PSA system. The I-stage PSA device separates out the CO+N₂ gas stream, and the II-stage PSA device separates out the H₂ gas stream.
Step 6: The CO+N₂ gas stream separated from the I-stage PSA device is pressurized to 0.5 MPa and introduced, as feedstock and diluent, into the DMO reactor for dimethyl oxalate synthesis. The reaction product is distilled and undergoes methanol recovery before being sent to the hydrogenation reaction.
Step 7: The H₂ gas stream separated from the II-stage PSA device reacts with the refined DMO in the EG synthesis reactor to produce ethylene glycol. The reaction product undergoes flash separation, methanol removal, and distillation to obtain the ethylene glycol product.

4. Processing Results:
Liquid carbon dioxide product composition and yield: CO₂ 99.9%, CO 0.1%; flow rate 80.11 t/h, pressure 2.5 MPa, temperature -12 °C.
Decarbonized gas composition and yield: CO 73.43%, H₂ 17.52%, N₂ 7.79%, CO₂ 0.92%, O₂ 0.33%; flow rate 62,256 Nm³/h, pressure 0.75 MPa, temperature 40 °C.
CO+N₂ gas stream composition and yield: CO 81.61%, N₂ 18.24%, CO₂ 0.07%; flow rate 16,242 Nm³/h (725.08 kmol/h), pressure 0.5 MPa, temperature 40 °C.
H₂ gas stream composition and yield: H₂ 99.99%, N₂ 0.01%; flow rate 12,292 Nm³/h (548.74 kmol/h), pressure 2.5 MPa, temperature 40 °C.
Hydrogen composition and supplementation: H₂ 99.99%, N₂ 0.01%; flow rate 13,621 Nm³/h (608.08 kmol/h), pressure 2.5 MPa, temperature 40 °C.

Ethylene glycol composition and yield: EG 99.9%, methyl acetate 0.1%; flow rate 11,943 kg/h (192.63 kmol/h), pressure 0.03 MPa, temperature 40 °C. Annual output 100,324 t (operation time assumed 8,400 h), corresponding to a design capacity of 100,000 t/year.

Overall, based on the above embodiments:
1. The embodiments of the present invention utilize PSA decarbonization combined with cooling, liquefaction, and distillation separation to produce high-purity liquid CO₂ that can be directly used for enhanced oil recovery, sequestration, or chemical synthesis, while achieving 100% recovery of the reducing components in blast furnace gas for blast furnace recycling and as chemical feedstock.
2. The embodiments of the present invention enable carbon cycling in an oxygen-blown blast furnace, where decarbonized gas is recycled back to the blast furnace, reducing coke rate and pulverized coal injection, thereby lowering carbon emissions per ton of steel.
3. The embodiments of the present invention separate CO+N₂ and H₂ for the synthesis of ethylene glycol using surplus decarbonized blast furnace gas, refined converter gas, and light hydrocarbon-converted coke oven gas through low-temperature methanol wash, temperature-swing adsorption, and two-stage PSA processes.
4. The embodiments of the present invention enable full utilization of the chemical energy of three types of steel plant gases, instead of only low-efficiency thermal utilization. By using surplus decarbonized gas, coke oven gas, and converter gas as chemical feedstocks, "carbon capture and emission reduction" can be achieved with added value, significantly reducing primary energy consumption at the source.
5. The embodiments of the present invention employ syngas produced without a gasification process, combined with CO₂ hydrogenation to methanol and catalytic coupling hydrogenation to ethylene glycol, saving the construction cost of gasification units and furnaces, effectively reducing the production cost of methanol and ethylene glycol, and fully utilizing renewable energy.
6. In the mixed gas purification process, the embodiments of the present invention do not separate part of N₂ from CO. This not only avoids the separation cost of CO and N₂ during purification but also reduces the gas required to reintroduce N₂ during DMO synthesis, effectively lowering ethylene glycol production cost.

It should be noted that the near-zero carbon emission blast furnace long-process steel integrated production system provided in the above embodiments and the near-zero carbon emission blast furnace long-process steel integrated production process belong to the same inventive concept. The specific operational modes of each unit and system have been described in detail in the process embodiments and are not repeated here. In practical application, the above process system may implement the described functions through different systems or devices according to specific requirements, either in whole or in part; the present application is not limited thereto.

The above embodiments are merely illustrative of the principles and effects of the present invention and are not intended to limit the present invention. Any person skilled in the art may make modifications or alterations to the above embodiments without departing from the spirit and scope of the present invention. Therefore, all equivalent modifications or alterations within the ordinary knowledge of the relevant technical field, which are made without departing from the spirit and technical concept disclosed by the present invention, shall still be encompassed by the claims of the present invention.

## Claims

1. A near-zero carbon emission integrated process for long-process blast furnace steelmaking and chemical co-production, **characterized by** comprising the following steps:
subjecting blast furnace gas to pressure swing adsorption decarbonization to separate a desorbed gas and a decarbonized gas;
subjecting the desorbed gas to liquefaction and rectification, thereby obtaining liquid carbon dioxide (liquid CO₂);
returning a portion of the decarbonized gas to the blast furnace, and mixing another portion of the decarbonized gas with purified converter gas and coke oven gas subjected to light hydrocarbon conversion to form a mixed gas;
subjecting the mixed gas stream to low-temperature methanol wash, temperature swing adsorption, and two-stage pressure swing adsorption, thereby separating two streams for ethylene glycol synthesis, wherein one stream comprises carbon monoxide (CO) and nitrogen (N₂), and the other stream comprises hydrogen (H₂).

2. The process of claim 1, wherein the liquid carbon dioxide is used for enhanced oil recovery, sequestration, or as a raw material for methanol synthesis; and/or
at least 80% of the decarbonized gas is recycled to the blast furnace, and not more than 20% of the decarbonized gas is mixed with the purified converter gas and the coke oven gas subjected to light hydrocarbon conversion to form the mixed gas; and/or
non-condensable gas separated during the rectification of the desorbed gas is used for cold energy recovery and then returned together with the blast furnace gas for pressure swing adsorption decarbonization to recover reductive components again.

3. The process of claim 1, wherein the decarbonized gas and the purified converter gas are subjected to catalytic desulfurization and deoxidation purification, and then mixed with the coke oven gas subjected to light hydrocarbon conversion to form the mixed gas.

4. The process of claim 1, wherein the process further comprises the following steps:
subjecting the pretreated blast furnace gas to pressure swing adsorption decarbonization,
subjecting the converter gas produced as a by-product in the converter steelmaking process to pre-treatment to obtain the purified converter gas; and
subjecting the coke oven gas after light hydrocarbon conversion to pretreatment, and then mixing with the decarbonized gas and the purified converter gas to form the mixed gas;
wherein the pre-treatment process comprises at least one procedure selected from dedusting, cooling, desulfurization, and pressurization.

5. The process of claim 1, wherein before the low-temperature methanol wash, in the mixed gas, the decarbonized gas, the purified converter gas, and the coke oven gas after light hydrocarbon conversion meet at least one of the following conditions (a) to (d):
(a) the temperature is less than or equal to 40°C;
(b) the oxygen content is less than or equal to 50 ppm;
(c) the hydrogen sulfide content is less than or equal to 0.1 ppm;
(d) the pressure is 2.0-3.5 MPa.

6. The process of claim 1, wherein after the low-temperature methanol wash, the mixed gas comprises H₂, CO, N₂, and other impurities;
wherein the impurities and their concentration requirements comprise: methanol ≤ 20 ppm, CO₂ ≤ 20 ppm, sulfides ≤ 0.1 ppm, oxygen ≤ 50 ppm; and/or
during the low-temperature methanol wash, the methanol absorbing impurities is subjected to methanol recovery treatment and then returned for recycling in the low-temperature methanol wash, while the separated hydrogen sulfide and CO₂ are introduced into the drying step of the desorbed gas for drying treatment, and subsequently subjected together with the desorbed gas to cryogenic liquefaction and rectification.

7. The process of claim 1, wherein:
after the low-temperature methanol wash, the mixed gas is subjected to temperature swing adsorption to remove heavy impurity components, wherein the heavy impurity components comprise methanol and moisture; and/or
after the temperature swing adsorption, the mixed gas is subjected to two-stage pressure swing adsorption to separate two streams, wherein the two-stage pressure swing adsorption comprises successively performed I-stage pressure swing adsorption and II-stage pressure swing adsorption, the stream separated by the I-stage pressure swing adsorption comprises CO and N₂, and the stream separated by the II-stage pressure swing adsorption comprises H₂.

8. The process of claim 1, wherein the synthesis of ethylene glycol adopts catalytic coupling hydrogenation technology, comprising the following steps:
using methyl nitrite as a raw material, and using the stream comprising CO and N₂ as a reactant gas and a diluent to synthesize dimethyl oxalate;
using the dimethyl oxalate synthesized in the preceding step, and using the stream comprising H₂ as a reactant gas to synthesize ethylene glycol; and/or
the process further comprises the step of supplementing hydrogen during the synthesis of ethylene glycol.

9. A near-zero carbon emission integrated system for long-process blast furnace steelmaking and chemical co-production, comprising:
a first unit, the first unit comprising a pressure swing adsorption decarbonization system for separating blast furnace gas into a desorbed gas and a decarbonized gas, and a liquefaction and rectification purification system for subjecting the desorbed gas to liquefaction and rectification purification to obtain liquid carbon dioxide;
a second unit, the second unit comprising a catalytic desulfurization and deoxidation system for subjecting the decarbonized gas and purified converter gas to catalytic desulfurization and deoxidation purification;
a third unit, the third unit comprising a light hydrocarbon conversion system for subjecting coke oven gas to light hydrocarbon conversion;
a fourth unit, the fourth unit comprising, arranged in sequence according to the process flow, a gas mixing system, a low-temperature methanol wash system, a temperature swing adsorption system, and a two-stage pressure swing adsorption system, wherein the gas mixing system is connected with the pressure swing adsorption decarbonization system and the light hydrocarbon conversion system, and is used for mixing the decarbonized gas, the purified converter gas, and the coke oven gas after light hydrocarbon conversion to form a mixed gas, and wherein the low-temperature methanol wash system, the temperature swing adsorption system, and the two-stage pressure swing adsorption system are sequentially used for subjecting the mixed gas to low-temperature methanol wash, temperature swing adsorption, and two-stage pressure swing adsorption to separate two streams for synthesizing ethylene glycol, wherein one stream comprises CO and N₂, and the other stream comprises H₂.

10. The system of claim 9, wherein the system further comprises at least one of the following units, systems, or equipment (1) to (11):
(1) the liquefaction and rectification purification system comprises a liquid carbon dioxide outlet, and the system further comprises a methanol synthesis unit connected with the liquid carbon dioxide outlet;
(2) the pressure swing adsorption decarbonization system comprises a decarbonized gas outlet, and the decarbonized gas outlet is connected with a blast furnace reducing gas inlet;
(3) the liquefaction and rectification purification system comprises rectification equipment and a precooler, wherein the rectification equipment comprises a non-condensable gas outlet connected with the precooler, the precooler uses the non-condensable gas as a cold source, and the precooler comprises a cold source outlet connected with an inlet of the pressure swing adsorption decarbonization system;
(4) the system further comprises a first pre-treatment system for pretreating blast furnace gas discharged from the blast furnace;
(5) the system further comprises a second pre-treatment system for pretreating converter gas produced as a by-product in the converter steelmaking process to obtain the purified converter gas;
(6) the system further comprises a third pre-treatment system arranged after a light hydrocarbon conversion system and used for pretreating the coke oven gas after light hydrocarbon conversion;
(7) the gas mixing system comprises pressurization device for pressurizing the mixed gas;
(8) the system further comprises a methanol recovery system for subjecting methanol absorbing impurities during the low-temperature methanol wash to methanol recovery treatment, wherein the liquefaction and rectification purification system comprises drying device for drying the desorbed gas, and the methanol recovery system is connected with the drying device so as to introduce hydrogen sulfide and CO₂ separated by the methanol recovery treatment into the drying device;
(9) the two-stage pressure swing adsorption system comprises, arranged in sequence according to the process flow, an I-stage pressure swing adsorption device and an II-stage pressure swing adsorption device; wherein the I-stage pressure swing adsorption device is used for subjecting the mixed gas after temperature swing adsorption to I-stage pressure swing adsorption to separate a stream comprising CO and N₂; and the II-stage pressure swing adsorption device is used for subjecting the mixed gas after the I-stage pressure swing adsorption to II-stage pressure swing adsorption to separate a stream comprising H₂;
(10) the system further comprises a dimethyl oxalate synthesis unit and an ethylene glycol synthesis unit;
wherein the dimethyl oxalate synthesis unit is connected with the two-stage pressure swing adsorption system and is used for synthesizing dimethyl oxalate by using the stream comprising CO and N₂ as a reactant gas,
and the ethylene glycol synthesis unit is connected with the dimethyl oxalate synthesis unit and the two-stage pressure swing adsorption system and is used for synthesizing ethylene glycol by using the dimethyl oxalate synthesized by the dimethyl oxalate synthesis unit as a raw material and using the stream comprising H₂ as a reactant gas;
(11) the system further comprises a hydrogen supplementation unit, the hydrogen supplementation unit being used for supplementing hydrogen during the process of synthesizing ethylene glycol.
